# EUROPÄISCHE PATENTANMELDUNG

(11) **EP 0 665 431 A2**
(43) Veröffentlichungstag der Anmeldung: **02.08.1995**
(21) Anmeldenummer: 94120481.0
(22) Anmeldetag: 23.12.1994
(51) Int. Cl.: G01N 27/48, G01N 33/22

(54) **Verfahren und Vorrichtung zur Detektion von Nitrotoluolen**

(30) Priorität: 14.01.1994 DE 4400859
(71) Anmelder: FRAUNHOFER-GESELLSCHAFT ZUR FÖRDERUNG DER ANGEWANDTEN FORSCHUNG E.V., D-80636 München (DE)
(72) Erfinder: Hambitzer, Günther, Dr., D-76327 Pfinztal (DE); Böke, Winfried, D-76229 Karlsruhe (DE)
(74) Vertreter: Dipl.-Ing. Heiner Lichti Dipl.-Phys. Dr.rer.nat. Jost Lempert Dipl.-Ing. Hartmut Lasch

(57) **Zusammenfassung**

Es wird ein Verfahren zur Detektion von Nitrotoluolen, insbesondere von Trinitrotoluol, Dinitrotoluol und Derivaten von Nitrotoluolen, in Böden, Flüssigkeiten und Gasen vorgeschlagen, wobei an eine Elektrode, die mit einer eine zu untersuchende Substanz aufweisenden Elektrolytlösung in Kontakt steht, eine Spannung angelegt wird, die Spannung zur Erzeugung einer Reduktionsreaktion verringert und dann zur Erzeugung einer Oxidationsreaktion erhöht wird und die Stromstärke zumindest eines Grundstromes und eines sich zeigenden anodischen Strommaximums insbesondere in Abhängigkeit von der angelegten Spannung bestimmt wird.

## Beschreibung

Die Erfindung betrifft ein Verfahren zur Detektion von Nitrotoluolen, insbesondere von Trinitrotoluol, Dinitrotoluol und Derivaten von Nitrotoluolen, in Böden, Flüssigkeiten und Gasen, sowie eine Vorrichtung zur Durchführung eines solchen Verfahrens.

Die Detektion und insbesondere die Bestimmung von Trinitrotoluol (TNT), Dinitrotoluol (DNT) und Nitrotoluolen (NT) sowie deren Derivaten spielt insbesondere bei der Untersuchung von Böden hinsichtlich der Feststellung von Rüstungsaltlasten auf dem Gelände bzw. der Umgebung von Sprengstoffbetrieben eine große Rolle. Obwohl häufig lediglich Flächen von einigen Quadratkilometern an einzelnen, unbekannten Stellen mit TNT-Nestern in der Größe von Quadratmetern verseucht sind, ist es bisher zumeist notwendig, auch den Boden nicht kontaminierter Flächen ganz abzutragen. Dies liegt insbesondere darin begründet, daß die bekannten Analysemethoden teuer sind und einen großen Zeitaufwand mit sich bringen. Dabei ist es notwendig, auf dem Gelände jeweils Proben zu entnehmen, die dann erst nach ca. einer Stunde an einem dafür vorgesehenen Analyseort analysiert werden können. Eine genaue Eingrenzung des kontaminierten Bereichs ist auf diese Weise nur mit einem hohen Zeit- und Arbeitsaufwand möglich. Auch bekannte Analysevorrichtungen für die TNT-Bestimmung benötigen für die Untersuchung einer Probe ca. eine Stunde. Da außerdem die Entsorgungskosten stark mit dem Volumen des verseuchten Aushubs steigen, ist entweder eine zeitaufwendige Bestimmung des genauen kontaminierten Bodenbereichs notwendig, oder aber es muß eventuell zuviel Boden mit den damit verbundenen Entsorgungskosten abgetragen werden.

Aber nicht nur die Untersuchung von Böden, sondern auch die von Flüssigkeiten hinsichtlich der Verseuchung mit TNT, DNT, NT und deren Derivaten ist notwendig, da Abwässer, Sickerwässer und Grundwässer durch Eluation von mit den genannten Substanzen verseuchten Böden bzw. durch die Reinigungsverfahren für die Böden belastet sein können. Auch hierfür sind derzeitig lediglich relativ zeitaufwendige und teure Verfahren, wie die Gaschromatographie, bekannt.

Des weiteren werden die meisten Sprengladungen wegen der technologisch einfachen Herstellung und Verarbeitbarkeit aus TNT oder mit Zusatz von TNT hergestellt. Da derartiger Sprengstoff auch bei erpresserischen oder terroristischen Drohungen sowie Anschlägen Verwendung findet, stellt die Auffindung derartiger Sprengstoffe, z.B. bei der Gepäck- und Passagierkontrolle im Flugverkehr, eine wichtige Aufgabe dar, um eine Gefährdung von Menschenleben zu verhindern. Auch bei der Minensuche von kunststoffummantelten Minen ist eine möglichst schnelle und unkomplizierte Detektion derartiger Minen unerläßlich.

Bei dem bei der Sprengstoffherstellung verwendeten TNT handelt es sich um einen Feststoff, der in kleinen Mengen Dampf an die Umgebung abgibt. Derzeitig ist dieser Dampf jedoch nur mit sehr aufwendigen Methoden, wie der Gaschromatographie mit Massenspektrometrie, detektierbar.

Der Erfindung liegt daher die Aufgabe zugrunde, ein Verfahren sowie eine Vorrichtung zur Bestimmung derartiger Kontaminationen in Böden, Flüssigkeiten und Gasen zu schaffen, die sich durch eine einfache, preiswerte und schnelle Detektion von TNT, DNT und NT vor Ort auszeichnen, wobei auch Kontaminationen unterschiedlicher Konzentrationen sicher und einfach in verschiedenartigsten Substanzen, wie Böden, Flüssigkeiten etc., detektiert werden können.

Erfindungsgemäß wird die genannte Aufgabe in einem Verfahren der eingangs genannten Art gelöst, welches dadurch gekennzeichnet ist, daß an eine Elektrode, die mit einer eine zu untersuchende Substanz aufweisenden Elektrolytlösung in Kontakt steht, eine Spannung angelegt, die Spannung zur Erzeugung einer Reduktionsreaktion verringert und dann zur Erzeugung einer Oxidationsreaktion erhöht wird und die Stromstärke zumindest eines Grundstromes und eines sich zeigenden anodischen Strommaximums insbesondere in Abhängigkeit von der angelegten Spannung bestimmt wird. Eine erfindungsgemäße Vorrichtung zeichnet sich dadurch aus, daß die Vorrichtung ein Sensorelement mit drei Elektroden, nämlich einer Arbeitselektrode, einer Bezugselektrode sowie einer Gegenelektrode aufweist und daß die Elektroden mit einer regelbaren Spannungsquelle verbunden sind.

Durch dieses erfindungsgemäße Verfahren sowie die erfindungsgemäße Vorrichtung können auch verschiedenartigste Böden, im subppm- bis 100 %-Bereich mit TNT, DNT kontaminiert, auf diese Stoffe hin untersucht werden, da die Nitrogruppen von DNT und TNT zunächst an der Arbeitselektrode reduziert werden. TNT, DNT sowie NT und ihre Derivate bilden jeweils für die jeweilige Kontamination spezifische Reduktionsprodukte aus, deren Oxidationsverhalten durch das Erhöhen der Spannung untersucht werden kann. Gleiches gilt für in Flüssigkeiten gelöste Kontaminationen sowie Dinitrotrotoluol (DNT) und Trinitrotoluol (TNT) in Gasen, die bei Raumtemperatur einen Gleichgewichtsdampfdruck von 55 700 bzw. 9400 ppt besitzen. Da eine derartige Vorrichtung sehr kompakt und tragbar ausbildbar ist, kann sie direkt vor Ort eingesetzt werden. Die Probeentnahme und -untersuchung, zwischen der beim Stand der Technik etwa eine Stunde liegt, entfällt.

Im Rahmen der Erfindung sind grundsätzlich alle Verfahren der Voltammetrie einsetzbar. In der Regel wird mit Gleichspannung gearbeitet, wobei die Stromstärke in Abhängigkeit von der eingestellten Spannung zwischen der Arbeitselektrode und einer Gegenelektrode bestimmt wird. Die Spannung wird dabei von einem Ausgangswert aus verringert, vorzugsweise kontinuierlich. Nach Erreichen eines unteren Wertes erfolgt dann wieder die ebenfalls vorzugsweise kontinuierliche Erhöhung. Hierbei erhält man sogenannte Voltammogramme in Form von Strom-Spannungs-Kurven, durch die festgestellt werden kann, ob eine Reduktion der Kontaminationen wie TNT, DNT oder NT sowie eine Oxidation ihrer Reduktionsprodukte stattgefunden hat.

Nach Erreichen eines festgelegten oberen Wertes wird die angelegte Spannung wiederum verringert, um eine Reduktionsreaktion an der Arbeitselektrode zu erzeugen. Hierdurch ist dann das Reduktionsverhalten des durch die Reduktionsprodukte jeweils ausgebildeten Redoxpaares durch Messen der Strom-Spannungs-Kurve bestimmbar geworden.

Nach Erreichen der Ausgangsspannung wird bevorzugt ein Meßzyklus abgeschlossen. Da die jeweils an der Arbeitselektrode auftretenden Stromspitzen für Reduktion und Oxidation der Redoxpaare für die unterschiedlichen Kontaminationen jeweils bei unterschiedlichen Spannungen liegen, ist durch deren Lage nach Abschluß eines Meßzyklus feststellbar, welche Kontamination(en) im Boden, in Flüssigkeiten sowie in Gasen enthalten sind. Durch die Höhe der Strom-Spannungs-Kurve bzw. der Fläche unterhalb der Strom-Spannungs-Kurve ist ein Maß für die Konzentration von DNT, TNT, NT sowie deren Derivaten gegeben. Demgemäß ist bei Durchführung des erfindungsgemäßen Verfahrens die Bestimmung dieser Kontaminationen problemlos durchführbar. Durch Festlegung des bei einem Meßzyklus durchfahrenen Spannungsbereiches sind Reaktionen von Störsubstanzen wie Kampfstoffen oder Abbauprodukten bzw. in Flüssigkeit gelösten Substanzen oder in Luft vorkommenden natürlichen oder künstlich hergestellten Gasen und Dämpfen ausschließbar. Das Verfahren zeichnet sich damit durch eine hohe Selektivität aus und weist keine Querempfindlichkeit gegenüber Störsubstanzen auf.

Eine äußerst bevorzugte Ausbildung des Verfahrens sieht vor, daß zunächst eine Spannung in einer Größe angelegt wird, bei der sich bei den zu bestimmenden Verbindungen ein Doppelschichtbereich ausbildet, daß die Spannung bis zu einem Umkehrpotential von mehr als 1 V erhöht wird, daß dabei die Stromstärke des sich im Doppelschichtbereich ergebenden Grundstroms gemessen wird, daß anschließend die Spannung zur Erzeugung einer Reduktionsreaktion verringert wird, daß dabei der Spannungswert eines sich ergebenden kathodischen Strommaximums bestimmt wird, daß weiter zur Erzeugung einer Oxidationsreaktion die Spannung wieder erhöht wird, daß dabei der Spannungswert bestimmt wird, bei dem sich ein anodisches Strommaximum ergibt, daß der Wert des anodischen Strommaximums bestimmt wird und daß der Quotient aus dem Wert des anodischen Strommaximums und dem gemessenen Wert des Grundstromes als für den Nitrotoluol-Gehalt der zu untersuchenden Substanz repräsentativer Wert bestimmt wird. Das anodische Strommaximum hat ein positives Vorzeichen, das kathodische ein negatives.

Die Ausgangsspannung wird bevorzugt mit ca. 500-900 mV, äußerst vorteilhaft bei ca. 700 mV, angelegt. Dieser Wert liegt im Doppelschichtbereich. Die Erhöhung erfolgt vorteilhafterweise bis zu einem Umkehrpotential der Meßelektrode von ca. 1,3 bis 1,5 V.

In bevorzugter Ausgestaltung kann vorgesehen sein, insbesondere in einem ersten Durchgang, daß nach Erreichen des kathodischen Strommaximus die Spannung um ca. weitere 300 mV reduziert wird.

Bevorzugt wird die Spannung über den Wert, bei dem sich das anodische Strommaximum ergibt, weiter erhöht und zwar insbesondere bis zum Ende des Doppelschichtbereichs. Es ist daher eine Erhöhung der Spannung um über ca. 700 mV über die Spannung, bei der sich das anodische Strommaximum zeigt, bzw. bis auf ca. 1300 mV vorteilhaft.

Die Spannungen werden insbesondere kontinuierlich oder quasi-kontinuierlich geändert. Das Potential wird in der Regel mit Geschwindigkeiten von zwischen 100 mV/sec und einigen V/sec, wie 5V/sec verändert.

Bevorzugt wird pro Sekunde ein Spannungsbereich von einem Volt abgefahren. Damit liegt der gesamte Arbeitsaufwand für einen Meßzyklus bei maximal 2 sec. Auf diese Weise ist es auch möglich, ein weites Gelände auf TNT, DNT, NT-haltige Nester mit einer Größenordnung von Quadratmetern einfach und schnell zu untersuchen. Es wird verhindert, daß eine zu große Menge an Boden abgetragen wird und hierdurch werden dann auch die Entsorgungskosten stark verringert, da eine Eingrenzung des kontaminierten Bereiches aufgrund der hohen Anzahl von pro Minute durchführbaren Messungen problemlos möglich ist.

Bevorzugt werden unterschiedliche Spannungsbereiche abgefahren. Auf diese Weise können mit Hilfe dieser schnellen zyklischen Messungen NT, DNT und TNT selektiv reduziert und nachgewiesen werden. Bevorzugt werden dabei Strom-Spannungs-Kurven unterschiedlicher Spannungsbereiche überlagert. Durch eine solche geeignete Überlagerung der Strom-Spannungs-Kurven in einem erweiterten Spannungsbereich, welcher die NT-Reduktion einschließt, mit den Strom-Spannungs-Kurven für die DNT- bzw. TNT-Bestimmung in den dafür abgefahrenen Spannungsbereichen, läßt sich NT in Gegenwart von DNT und TNT quantitativ nachweisen. Weiterbildungen sehen vor, daß nach einem Meßzyklus die Elektroden zum Reinigen der Arbeitselektrode mehrfach umgepolt werden. Durch das schnelle Schalten zwischen Sauerstoff- und Wasserstoffentwicklung wird die sich an der Arbeitselektrode bildende Schicht entfernt. Alternativ oder in Kombination mit dem mehrfachen Umpolen ist vorgesehen, daß die Oberfläche der Arbeitselektrode periodisch nach einem Meßzyklus oder aber permanent bevorzugt durch einen mechanischen Schaber oder Schleifer gesäubert wird. Eine Kombination beider Reinigungsverfahren bietet sich vorzugsweise dann an, wenn hohe Konzentrationen an TNT oder DNT bzw. desaktivierenden Störsubstanzen in der zu untersuchenden Substanz enthalten sind. Ohne eine derartige Säuberung würden die Elektroden desaktiviert und die Höhe der Stromspitzen abnehmen, wodurch eine Verfälschung der Meßergebnisse eintreten würde.

Um das erfindungsgemäße Verfahren bei einer Substanz durchzuführen, deren pH-Wert nicht niedrig genug ist, wird der Umgebungsbereich der Arbeitselektrode angesäuert. Für kontinuierliche Messungen ist weiterbildend vorgesehen, daß eine zu untersuchende flüssige Substanz am Elektrodensystem mit der Arbeitselektrode vorbeigeführt wird. Hierzu wird bevorzugt die flüssige Substanz durch eine Durchflußzelle geleitet.

Da DNT und TNT einen Gleichgewichtsdampfdruck von 55700 bzw. 9400 ppt bei Raumtemperatur aufweisen, ist für die selektive Bestimmung dieser Stoffe sowie NT und deren Derivate bis in den ppt-Bereich vorgesehen, daß eine gasförmige Substanz an einer flüssigkeitsundurchlässigen, aber gasdurchlässigen Membran vorbeigeführt wird, durch die die gasförmige Substanz in die Elektrolytlösung gelangt. Bevorzugt wird dabei die gasförmige Substanz durch Pumpen an der Membran vorbeigeführt. Hochentwickelte, komplizierte Apparaturen sowohl hinsichtlich der Mechanik und Sensorik als auch hinsichtlich der Elektronik, die für die aus dem Stand der Technik bekannte Gaschromatographie notwendig sind, sind nicht mehr erforderlich. Auch ist eine kontinuierliche Messung durchführbar.

Um den Übergang des TNT bzw. DNT sowie NT etc. aus der Gasphase in die flüssige Phase zu erhöhen, wird der Elektrolytlösung bevorzugt ein schwerflüchtiges Co-Lösungsmittel zugesetzt. Um die Reaktionsgeschwindigkeit und damit die Ansprechzeit zu erhöhen, wird die Elektrolytlösung vor der Durchführung eines Meßzyklus bevorzugt erhitzt.

Die Bildung des TNT-Reduktionsprodukts und der Verlauf des TNT-Reduktionsstrom unabhängig von der Zusammensetzung der zu untersuchenden Substanz sowie der eindeutige Zusammenhang zwischen TNT-Reduktion und Oxidation des Reduktionsprodukts, charakterisiert durch Höhe und Potentiallage des anodischen Strompeaks, sind nicht trivial und nicht vorhersehbar und daher unerwartet und überraschend.

Der bei Potentialen unterhalb von 500 mV gemessene Strom ist im allgemeinen eine Überlagerung von Strömen, die auf die Reduktion, u.U. auch Oxidation von elektrochemisch reaktionsfähigen, nach Art und Konzentration unbekannten Substanzen zurückzuführen sind. Eine direkte Messung des TNT-Reduktionsstroms als Maß für die TNT-Konzentration ist daher nicht möglich.

Erfindungsgemäß ist weiterhin die Verwendung des vorgenannten Verfahrens zur Detektion von Nitrotoluolen, insbesondere Trinitrotoluol, Dinitrotoluol und Derivaten von Nitrotoluolen, in Böden, Flüssigkeiten und Gasen vorgesehen.

Bei der Arbeitselektrode handelt es sich bevorzugt um eine Elektrode, die zumindest Gold enthält. Eine mögliche Ausführungsform für die Arbeitselektrode ist dabei die Ausführung als Doppelstiftelektrode, eine andere mögliche Ausführungsform ist die Ausführung als Ringelektrode.

Bei der Bezugselektrode handelt es sich bevorzugt um eine Platinelektrode, die durch Zuführung oder durch kathodische Entwicklung von Wasserstoff die Eigenschaften einer reversiblen Wasserstoffelektrode hat. Als Ausführungsformen der Bezugselektrode ist diejenige als Platindraht oder eine als Platinstift möglich.

Weiterbildungen sehen vor, daß die Elektroden in eine Elektrolytlösung eintauchen, in der die zu untersuchende Substanz gelöst ist. Dies ist zur Durchführung des erfindungsgemäßen Verfahrens notwendig. Bei der Elektrolytlösung handelt es sich bevorzugt um Schwefel- oder aber um Phosphorsäure. Durch diese Säuren werden die genannten Gold- und Platinelektroden nicht angegriffen.

Zur Untersuchung von flüssigen und gasförmigen Substanzen sehen Weiterbildungen vor, daß unterhalb des Sensorelementes eine Durchflußzelle angeordnet ist. Auf diese Weise sind kontinuierliche Messungen an flüssigen sowie gasförmigen Substanzen möglich. Diese Durchflußzelle ist bevorzugt derart ausgebildet, daß sie auf das Sensorelement aufschraubbar ist. Damit ist ein Einsatz desselben Sensorelementes in unterschiedlichen Umgebungen und für unterschiedliche Substanzen, nämlich Böden, Gas und Flüssigkeiten, möglich. Die Durchflußzelle wird dann bei Bedarf auf das Sensorelement aufgeschraubt. Auf diese Weise sind Kontaminationen in Böden, Flüssigkeiten und Gasen mit lediglich einer Vorrichtung selektiv und zuverlässig meßbar.

Weiterbildungen sehen vor, daß für die Untersuchung von Gasen hinsichtlich einer Kontamination die Durchflußzelle durch eine flüssigkeitsundurchlässige, aber gasdurchlässige Membran von dem Sensorelement und der Elektrolytlösung getrennt ist. Auf diese Weise kann zwar das Gas in die Elektrolytlösung eindringen, ein Austritt der Elektrolytflüssigkeit in die Durchflußzelle wird jedoch verhindert. Die Porengröße beträgt bevorzugt 40 nm, und direkt an der der Durchflußzelle abgewandten Seite der Poren ist die Arbeitselektrode angeordnet.

Weiterbildungen sehen vor, daß zum Pumpen des Gases durch die Durchflußzelle eine Pumpeinrichtung vorgesehen ist. Hierdurch kann auch eine kontinuierliche Messung erfolgen.

In weiterer bevorzugter Ausgestaltung ist vorgesehen, daß das Sensorsystem eine Regelungs- und Auswerteelektronik aufweist. Auf diese Weise können aus den bei den angelegten Spannungswerten gemessenen Stromwerten die Konzentrationen von TNT, DNT und NT sowie deren Derivaten bestimmt werden. Bevorzugt ist eine Ausgabeeinheit zur Konzentrationsanzeige vorgesehen, so daß direkt beim Messen die Konzentration abgelesen werden kann. In Weiterbildung ist vorgesehen, daß die Vorrichtung eine Alarmeinheit zur Ausgabe eines Alarmsignals bei Überschreiten eines vorgegebenen Konzentrationswertes aufweist.

Bevorzugt ist eine Einrichtung zum Reaktivieren zumindest der Arbeitselektrode vorgesehen. Als solche kann unterhalb der Elektroden ein Schaber oder Schleifer zum Reinigen der Elektrodenoberfläche vorgesehen sein.

Durch den Schaber bzw. Schleifer wird dafür gesorgt, daß die Arbeitselektrode zur Erzielung reproduzierbarer Meßergebnisse immer wieder auf einfache Weise gereinigt werden kann. Dies ist notwendig, da sie durch die Messung desaktiviert wird und sich eine Schicht an der Elektrode bildet, die entfernt werden muß.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnungen im einzelnen erläutert ist:
- Figur 1: eine Strom-Spannungs-Kurve für in Schwefelsäure gelöstes Trinitrotoluol (TNT) bei Messung in einer herkömmlichen Meßzelle;
- Figur 2: eine Strom-Spannungs-Kurve für in Schwefelsäure gelöstes Trinitrotoluol (TNT), gemessen mit einer Goldmikroelektrode; und
- Figur 3: eine schematische Darstellung eines Sensorelementes der erfindungsgemäßen Vorrichtung.

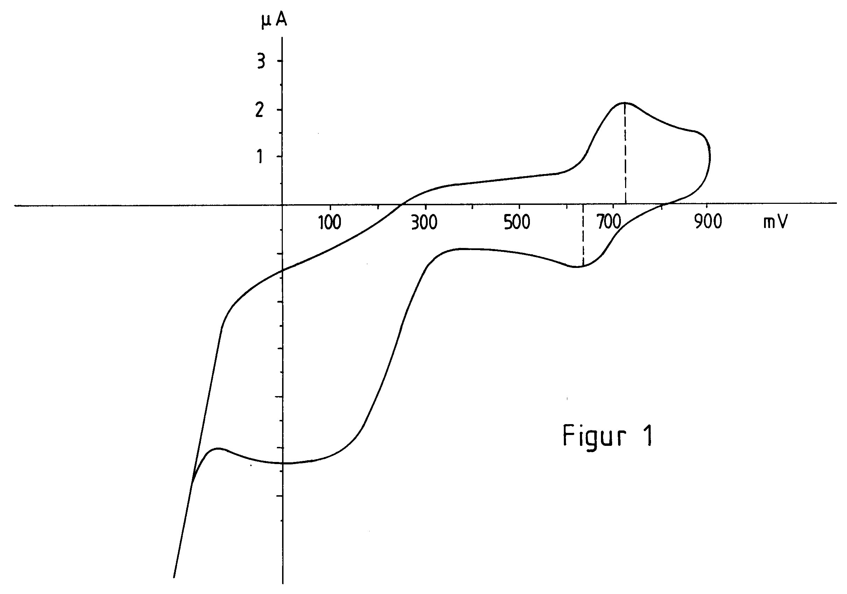

Bei einem konkreten Beispiel des erfindungsgemäßen Verfahrens wurden 50 mg/l TNT in 10-n Schwefelsäure (H₂SO₄) gelöst und die Stromstärke in Abhängigkeit von der angelegten Spannung in einem Spannungsbereich von etwa 0 bis 1000 mV gemessen. In Figur 1 ist die Strom-Spannungs-Kurve für 50 mg/l TNT in 10-n Schwefelsäure dargestellt.

Anodische Ströme, die elektrochemischen Oxidationsreaktionen entsprechen, sind in positiver Y-Richtung, kathodische Ströme, die elektrochemischen Reduktionen entsprechen, in negativer Y-Richtung aufgezeichnet. Alle Spannungs- bzw. Potentialangaben der Strom-Spannungs-Kurve (Voltammogramm) beziehen sich auf das Potential der Normalwasserstoffelektrode.

Der vorgegebene Spannungsbereich wird während des erfindungsgemäßen Verfahrens mit 1 Volt/sec. abgefahren, so daß ein Meßzyklus maximal 2 sec. dauert.

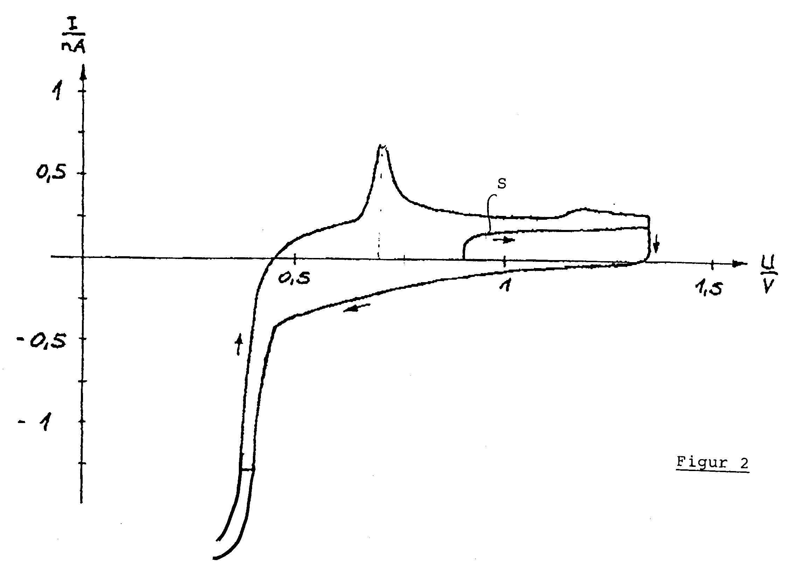

Die Figur 1 zeigt das Ergebnis eines Demonstrationsversuchs mit einer herkömmlichen Halbzelle, bei der die Meßelektrode ein ca. 1 cm² großes Blech war. Die an einer Arbeitselektrode 13 (s. Figur 2) angelegte Ausgangsspannung von etwa 500 mV wird zunächst auf etwa 100 mV verringert, und der zwischen der Arbeitselektrode und einer Gegenelektrode 14 (s. Figur 2) jeweils fließende Strom wird in Abhängigkeit von der angelegten Spannung gemessen. Bei Verringerung der Ausgangsspannung von etwa 500 mV auf 100 mV tritt die elektrochemische Reduktion von TNT ein. Dabei werden die Nitrogruppen des TNT reduziert und es bilden sich Reduktionsprodukte, auf die im folgenden noch näher eingegangen wird. Das Einsetzen der Reduktion von TNT zeigt sich am stark anwachsenden kathodischen Strom unterhalb von etwa 500 mV, hier mit einem Strommaximum bei etwa 100 mV. Das kathodische Strommaximum ist umgebungsabhängig.

Nach Erreichen des unteren Spannungswertes wird die Spannung in 1 V/sec.-Schritten auf etwa 900 mV erhöht. Wie aus dem Voltammogramm in Figur 1 ersichtlich ist, weist die Strom-Spannungs-Kurve bei etwa 700 mV für 50 mg/l TNT eine anodische Stromspitze auf. Bei dieser Spannung erfolgt eine Oxidation des Reduktionsproduktes.

Nach Erreichen des oberen Spannungswertes von 900 mV wird die Spannung an der Arbeitselektrode wiederum verringert. Dabei zeigt die Strom-Spannungs-Kurve bei etwa 600 mV eine kathodische Stromspitze. In diesem Spannungsbereich hat eine Reduktion des durch Oxidation aus den Reduktionsprodukten gebildeten Reaktionsprodukten stattgefunden. Für Spannungen unterhalb von etwa 500 mV setzt wiederum eine Reduktion der Nitrogruppen des TNT ein.

Das Potentialintervall, in dem zur Bestimmung des Nitrotoluols gearbeitet wird, erstreckt sich von einem unteren, kathodischen Umkehrpotential, das bis zu 300 mV negativer ist als das Potential des kathodischen Strommaximums, zu einem oberen, anodischen Umkehrpotential, das bis zu ca. 700 mV positiver ist als das Potential des anodischen Strommaximums.

Das bei der Reduktion der Nitrogruppen von TNT gebildete Reduktionsprodukt bildet, wie aus dem Voltammogramm ersichtlich ist, ein durch die anodischen und kathodischen Stromspitzen gut nachweisbares Redoxpaar aus, über das dann der Nachweis von TNT auf einfache und schnelle Weise direkt vor Ort erfolgen kann.

Die Stromspitzen des Redoxpaares weisen für unterschiedliche TNT-Konzentrationen eine unterschiedliche Höhe auf.

Die Fläche unter der Strom-Spannungs-Kurve bzw. deren Höhe stellt ein Maß für die Konzentration des TNT in der Schwefelsäure dar. Um die Meßgenauigkeit zu erhöhen, können unterschiedliche Spannungsbereiche abgefahren werden.

Die Strom-Spannungs-Kurven für DNT sowie NT und deren Derivate weisen entsprechende Verläufe auf. Jedoch setzt die Reduktion von DNT, NT etc. bei niedrigeren Spannungswerten als bei TNT ein. Auch die Redoxpaare bzw. Redoxsysteme weisen gegenüber dem Redoxpaar von TNT bezüglich der Spannung verschobene Stromspitzen auf. Durch eine geeignete Überlagerung der Strom-Spannungs-Kurven in erweiterten Spannungsbereichen läßt sich NT in Gegenwart von DNT und TNT quantitativ nachweisen. Statt Schwefelsäure ist auch Phosphorsäure als Elektrolyt verwendbar. TNT-ähnliche Verbindungen wie Toluol- bzw. Xylolmoschus sowie die verschiedenen Aminotoluole weisen in den gewählten Spannungsbereichen keine Reaktion auf, so daß keine Querempfindlichkeit besteht. Auch ist das Verfahren für Temperaturbereiche von 0 °C bis 40 °C verwendbar, ohne daß sich eine Verfälschung der Meßergebnisse ergibt.

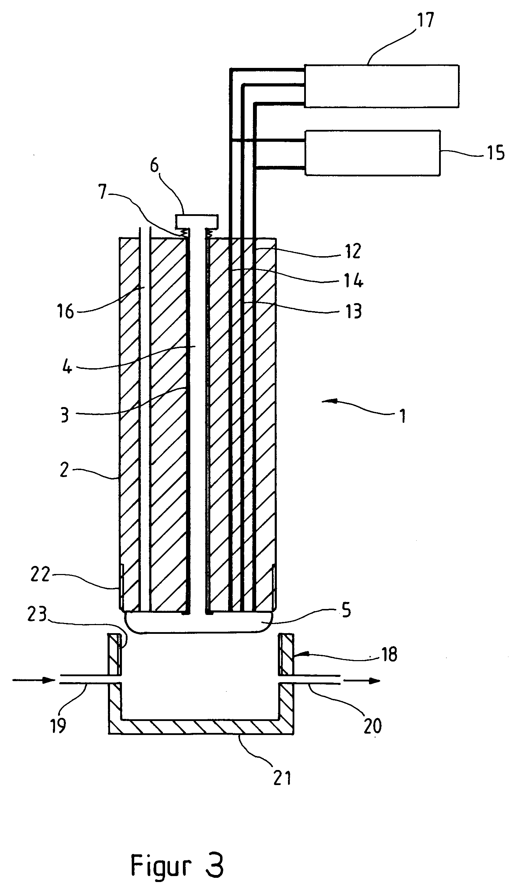

Die Fig. 2 zeigt die TNT-Bestimmung mit der gleichen Lösung wie nach Fig. 1, allerdings mit einer Goldmikroelektrode in Form eines Drahtes mit einem Durchmesser von ca. 25 µm in einem Sensorelement nach Figur 3. Es ergeben sich wesentlich geringere Ströme (bei allerdings höherer Stromdichte). Die Messung beginnt hier im Doppelschichtbereich S der Meßelektrode bei einem Startpotential zwischen 700 mV bis 1000 mV. Von hier aus wird das Potential der Meßelektrode (Arbeitselektrode) mit einer Geschwindigkeit von ca. 200 mV/sec bis zum oberen Umkehrpotential verändert. Der in dieser Startphase fließende Strom ist ausschließlich der Umladestrom der elektrochemischen Doppelschicht (kapazitiver Strom).

Vom oberen Umkehrpunkt wird das Potential in negativer Richtung bis zum unteren Umkehrpunkt (kathodischer Lauf) und wieder zurück zum oberen Umkehrpunkt (anodischer Lauf) mit derselben Spannungsgeschwindigkeit durchlaufen. Damit ist ein Meßzyklus abgeschlossen. Die TNT-Reduktion erfolgt in einem Potentialbereich kleiner 500 mV, in dem eine Vielzahl von Substanzen, die in unbekannten Böden, Flüssigkeiten oder Gasen enthalten sein können, ebenfalls elektrochemisch reduziert werden, wobei der infolge der TNT-Reduktion fließende Strom im Potentialbereich von 400 bis 100 mV RHE ein charakteristisches Maximum (kathodisches Strommaximum) aufweist.

Der Quotient aus dem maximalen Strom (anodischer Strompeak) beim anodischen Lauf und dem in der Startphase gemessenen Strom (kapazitiver Strom) ist dem TNT-Gehalt proportional.

Ein in Figur 3 dargestelltes Sensorelement 1 weist ein Gehäuse 2 auf, welches in seinem Zentrum mit einer Bohrung 3 versehen ist. In diese Bohrung 3 ist ein Stiel 4 eingelassen, an dessen unterem, mit dem Gehäuse 2 schlüssig abschließenden Ende ein Schaber 5 angeordnet ist. An dem dem Schaber 5 entgegengesetzten Ende weist der aus dem Gehäuse 2 herausragende Stiel 4 einen Kopf 6 auf, an dessen Unterseite zwischen Kopf 6 und Gehäuse 2 eine Feder 7 angeordnet sein kann. Die Feder 7 gewährleistet, daß der Schaber immer an der Gehäuseunterseite anliegt.

Durch die Gehäusehälfte 8 des Gehäuses 2 sind Elektroden 12, 13, 14 geführt. Dabei handelt es sich um eine Bezugselektrode 12, eine Arbeitselektrode 13 sowie eine Gegenelektrode 14. Bei der Bezugselektrode 12 handelt es sich um eine Wasserstoffelektrode, die im dargestellten Ausführungsbeispiel ein mit kathodischem Strom über eine Konstantstromquelle 15 belasteter Platinstift ist. Durch die kathodische Gleichstrombelastung von ca. 1 mA/cm² wird an der Bezugselektrode 12 Wasserstoff entwickelt. Da das hierdurch erzeugte System Wasserstoff/ Bezugselektrode (Platinstift) eine sehr geringer Überspannung und hohe Austauschstromdichte aufweist, stellt sich trotz kathodischer Polarisation der Bezugselektrode 12 nahezu das reversible Wasserstoffpotential ein. Die Arbeitselektrode 13, mit der das elektrochemische Verhalten untersucht werden soll, ist ein Goldstift und mit der Gegenelektrode 14 über ein Mikroamperemeter (nicht dargestellt) mit einer ebenfalls nicht dargestellten Spannungsquelle, bevorzugt einer Gleichspannungsquelle einer Regelungs- und Auswerteeinheit 17 verbunden, deren Spannung meßbar verändert werden kann. Bei der Gegenelektrode 14 handelt es sich ebenfalls um eine Goldelektrode. Ein spezielles Regelgerät, z.B. ein Potentiostat, regelt den Strom zwischen Arbeits- und Gegenelektrode in der Weise, daß sich die gewünschte Potentialdifferenz zwischen Arbeits- und Bezugselektrode einstellt. Die Energieversorgung der Vorrichtung kann über eine mobile Versorgungseinheit, z.B. Batterien, erfolgen, so daß die Tragfähigkeit der Vorrichtung gewährleistet bleibt.

Als weitere Bauform bietet sich die bekannte Mikroelektrode an, deren Oberfläche vorzugsweise ebenfalls mechanisch bearbeitet wird.

In der den Durchführungen für die Elektroden 12-14 gegenüberliegenden Längsseite des Gehäuses 2 ist eine Zuführleitung 16 für ein Elektrolyt angeordnet. Bei dem Elektrolyt handelt es sich bevorzugt um Schwefel- oder Phosphorsäure (H₂SO₄; H₃PO₄). Durch diese Säuren werden die Gold- bzw. Platinelektroden 12, 13, 14 nicht angegriffen.

Das Sensorelement 1 ist zur Bestimmung von TNT, DNT, NT sowie deren Derivaten mittels des erfindungsgemäßen Verfahrens in eine Durchflußzelle 18 eintauchbar. Die Durchflußzelle 18 weist einen Einlaß 19 sowie einen Auslaß 20 auf, durch die zu untersuchende flüssige oder gasförmige Substanzen durch die Durchflußzelle 18 hindurch am Sensorelement 1 vorbeigeführt werden können. Um dasselbe Sensorelement 1 in verschiedenen Umgebungen und für unterschiedliche Substanzen (Böden, Flüssigkeiten, Gase) einsetzen zu können, sind das Gehäuse 2 des Sensorelementes 1 an seiner Außenseite im unteren Bereich des Gehäuses 2 und das Gehäuse 21 der Durchflußzelle 18 an seiner Innenseite jeweils mit Windungen 22, 23 versehen. Auf diese Weise kann die oben offen ausgebildete Durchflußzelle 18 je nach Bedarf auf das Sensorelement 1 aufgeschraubt werden.

Für die Untersuchung von Gasen kann unterhalb des Sensorelementes 1 eine nanoporöse, hydrophobe Membran angeordnet sein, wobei auf der dem Sensorelement abgewandten Seite der Membran die Durchflußzelle 18 für das Gas angeordnet ist, durch die das Gas mittels einer Pumpe vorbeigepumpt wird. Die Arbeitselektrode 12 ist dabei bevorzugt an den Enden der Poren der Membran angeordnet. Die Größe der Poren beträgt bevorzugt 40 nm. Um den Übergang des TNT/DNT aus der Gasphase in die flüssige Phase zu erhöhen, wird die Elektrolytlösung mit einem schwerflüchtigen, TNT/DNT gut lösenden organischen Co-Lösungsmittel gemischt.

Die Elektroden 12, 13, 14 des Sensorelementes 1 sind gemeinsam mit der Regel- sowie Auswerteelektronik 17 verbunden, über die aus den angelegten Spannungs- und den gemessenen Stromwerten die Konzentrationen von TNT, DNT und NT sowie deren Derivaten bestimmt werden können. Über eine Ausgabeeinheit können die Konzentrationen direkt abgelesen werden. Auch kann eine Alarmeinheit zur Ausgabe eines Alarmsignals bei Überschreitung eines Schwellwertes bezüglich der Konzentration vorgesehen sein.

Insgesamt sind also ein Verfahren und eine Vorrichtung geschaffen, mittels derer Kontaminationen wie TNT, DNT, NT und deren Derivaten in Böden, Flüssigkeiten sowie Gasen preiswert, einfach und schnell selektiv bestimmt werden können. Die Vorrichtung ist vor Ort einsetzbar, so daß die bisherigen Zeiten von einer Stunde zwischen Probenentnahme und -bestimmung entfallen. Der kontaminierte Bereich kann genau eingegrenzt werden, so daß die Entsorgungskosten reduziert werden können. Des weiteren ist die Vorrichtung auch noch nach mehrjähriger Lagerdauer einsetzbar. Hierdurch wird die Sicherheit der sie verwendenden Personen, insbesondere bei der Minensuche, gewährleistet.

## Patentansprüche

1. Verfahren zur Detektion von Nitrotoluolen, insbesondere von Trinitrotoluol, Dinitrotoluol und Derivaten von Nitrotoluolen, in Böden, Flüssigkeiten und Gasen, dadurch gekennzeichnet,
daß an eine Elektrode, die mit einer eine zu untersuchende Substanz aufweisenden Elektrolytlösung in Kontakt steht, eine Spannung angelegt wird, die Spannung zur Erzeugung einer Reduktionsreaktion verringert und dann zur Erzeugung einer Oxidationsreaktion erhöht wird und die Stromstärke zumindest eines Grundstromes und eines sich zeigenden anodischen Strommaximums insbesondere in Abhängigkeit von der angelegten Spannung bestimmt wird.

2. Verfahren nach Anspruch 1, dadurch gekennzeichnet, daß die angelegte Spannung nach Erreichen eines festgelegten oberen Wertes verringert wird, um eine Reduktionsreaktion zu erzeugen.

3. Verfahren nach Anspruch 2, dadurch gekennzeichnet, daß nach Erreichen der Ausgangsspannung ein Meßzyklus abgeschlossen wird.

4. Verfahren nach einem der Ansprüche 1 bis 3, dadurch gekennzeichnet,
- daß zunächst eine Spannung in einer Größe - bezogen auf eine Wasserstoffelektrode - angelegt wird, bei der sich bei den zu bestimmenden Verbindungen ein Doppelschichtbereich ausbildet,
- daß die Spannung bis zu einem Umkehrpotential von mehr als 1 V erhöht wird,
- daß dabei die Stromstärke des sich im Doppelschichtbereich ergebenden Grundstroms gemessen wird,
- daß anschließend die Spannung zur Erzeugung einer Reduktionsreaktion verringert wird,
- daß dabei der Spannungswert eines sich ergebenden kathodischen Strommaximums bestimmt wird,
- daß weiter zur Erzeugung einer Oxidationsreaktion die Spannung wieder erhöht wird;
- daß dabei der Spannungswert bestimmt wird, bei dem sich ein anodisches Strommaximum ergibt,
- daß die Stromstärke des anodischen Strommaximums bestimmt wird, und
- daß der Quotient aus der Stromstärke des anodischen Strommaximums und der gemessenen Stromstärke des Grundstromes als für den Nitrotoluol-Gehalt der zu untersuchenden Substanz repräsentativer Wert bestimmt wird.

5. Verfahren nach einem der Ansprüche 1 bis 4, dadurch gekennzeichnet, daß die Spannung über den Wert hinaus, bei dem sich ein anodisches Strommaximum ergibt, weiter erhöht wird.

6. Verfahren nach einem der Ansprüche 1 bis 5, dadurch gekennzeichnet, daß unterschiedliche Spannungsbereiche abgefahren werden.

7. Verfahren nach Anspruch 6, dadurch gekennzeichnet, daß die Strom-Spannungs-Kurven unterschiedlicher Spannungsbereiche überlagert werden.

8. Verfahren nach einem der Ansprüche 1 bis 7, dadurch gekennzeichnet, daß nach einem Meßzyklus die Elektroden zum Reinigen der Arbeitselektrode mehrfach umgepolt werden.

9. Verfahren nach einem der Ansprüche 1 bis 8, dadurch gekennzeichnet, daß die Oberfläche der Arbeitselektrode periodisch nach einem Meßzyklus gesäubert wird.

10. Verfahren nach Anspruch 9, dadurch gekennzeichnet, daß die Oberfläche der Arbeitselektrode durch einen mechanischen Schaber oder Schleifer gereinigt wird.

11. Verfahren nach einem der Ansprüche 1 bis 10, dadurch gekennzeichnet, daß die Oberfläche der Arbeitselektrode permanent durch einen Schaber oder Schleifer gesäubert wird.

12. Verfahren nach einem der Ansprüche 1 bis 11, dadurch gekennzeichnet, daß der Umgebungsbereich der Arbeitselektrode angesäuert wird.

13. Verfahren nach Anspruch 12, dadurch gekennzeichnet, daß eine zu untersuchende flüssige Substanz an einem Sensorelement mit der Arbeitselektrode vorbeigeführt wird.

14. Verfahren nach Anspruch 13, dadurch gekennzeichnet, daß die flüssige Substanz durch eine Durchflußzelle geleitet wird.

15. Verfahren nach einem der Ansprüche 1 bis 13, dadurch gekennzeichnet, daß eine gasförmige Substanz an einer flüssigkeitsundurchlässigen, aber gasdurchlässigen Membran vorbeigeführt wird, durch die die gasförmige Substanz in die Elektrolytlösung gelangt.

16. Verfahren nach Anspruch 15, dadurch gekennzeichnet, daß die gasförmige Substanz durch Pumpen an der Membran vorbeigeführt wird.

17. Verfahren nach Anspruch 15 oder 16, dadurch gekennzeichnet, daß der Elektrolytlösung ein schwerflüchtiges Co-Lösungsmittel zum Lösen von Trinitrotoluol bzw. Dinitrotoluol zugesetzt wird.

18. Verfahren nach einem der vorangehenden Ansprüche, dadurch gekennzeichnet, daß die Elektrolytlösung vor der Durchführung eines Meßzyklus erhitzt wird.

19. Verwendung eines Verfahrens nach einem der Ansprüche 1 bis 18 zur Bestimmung von Kontaminationen, insbesondere Trinitrotoluol, Dinitrotoluol und Derivaten von Nitrotoluolen in Böden, Flüssigkeiten und Gasen.

20. Vorrichtung zur Detektion von Nitrotoluolen, insbesondere von Trinitrotoluol, Dinitrotoluol und Derivaten von Nitrotoluolen in Böden, Flüssigkeiten und Gasen, insbesondere zur Durchführung des Verfahrens nach einem der vorangehenden Ansprüche, dadurch gekennzeichnet, daß die Vorrichtung ein Sensorelement (1) mit drei Elektroden (12, 13, 14), nämlich einer Arbeitselektrode (13), einer Bezugselektrode (12) sowie einer Gegenelektrode (14), aufweist und daß die Elektroden mit einer regelbaren Spannungsquelle (17) verbunden sind.

21. Vorrichtung nach Anspruch 20, dadurch gekennzeichnet, daß die Arbeitselektrode (13) zumindest Gold enthält.

22. Vorrichtung nach Anspruch 20 oder 21, dadurch gekennzeichnet, daß die Arbeitselektrode (13) eine Doppelstiftelektrode ist.

23. Vorrichtung nach einem der Ansprüche 20 oder 21, dadurch gekennzeichnet, daß die Arbeitselektrode (13) eine Ringelektrode ist.

24. Vorrichtung nach einem der Ansprüche 20 bis 23, dadurch gekennzeichnet, daß die Bezugselektrode (12) aus Platin, insbesondere in Form eines Platindrahtes oder -stiftes, ist.

25. Vorrichtung nach einem der Ansprüche 20 bis 24, dadurch gekennzeichnet, daß die Elektroden (12, 13, 14) in eine Elektrolytlösung eintauchen, in der die zu untersuchende Substanz gelöst ist.

26. Vorrichtung nach Anspruch 25, dadurch gekennzeichnet, daß die Elektrolytlösung Schwefel- oder Phosphorsäure ist.

27. Vorrichtung nach einem der Ansprüche 20 bis 26, dadurch gekennzeichnet, daß unterhalb des Sensorelementes (1) eine Durchflußzelle (18) angeordnet ist.

28. Vorrichtung nach Anspruch 27, dadurch gekennzeichnet, daß die Durchflußzelle (18) auf dem Sensorelement (1) zu befestigen ist.

29. Vorrichtung nach Anspruch 27 oder 28, dadurch gekennzeichnet, daß die Durchflußzelle (18) durch eine flüssigkeitsundurchlässige, aber gasdurchlässige Membran von dem Sensorelement (1) und der Elektrolytlösung getrennt ist.

30. Vorrichtung nach Anspruch 29, dadurch gekennzeichnet, daß die Membran Poren mit einer Größe von kleiner als 1 µm aufweist.

31. Vorrichtung nach Anspruch 30, dadurch gekennzeichnet, daß an der der Durchflußzelle (18) abgewandten Seite direkt an den Poren die Arbeitselektrode (14) angeordnet ist.

32. Vorrichtung nach einem der Ansprüche 29 bis 31, gekennzeichnet durch eine Pumpeinrichtung zum Pumpen des Gases durch die Durchflußzelle (18).

33. Vorrichtung nach einem der Ansprüche 20 bis 32, dadurch gekennzeichnet, daß das Sensorsystem (1) eine Regelungs- und Auswerteelektronik (17) aufweist.

34. Vorrichtung nach einem der Ansprüche 20 bis 33, dadurch gekennzeichnet, daß die Vorrichtung eine Ausgabeeinheit zur Konzentrationsanzeige aufweist.

35. Vorrichtung nach einem der Ansprüche 20 bis 34, dadurch gekennzeichnet, daß die Vorrichtung eine Alarmeinheit zur Ausgabe eines Alarmsignals bei Überschreiten eines vorgegebenen Konzentrationswertes an Trinitro-, Dinitro-, Nitrotoluol sowie deren Derivaten aufweist.

36. Vorrichtung nach einem der Ansprüche 20 bis 35, dadurch gekennzeichnet, daß eine Einrichtung (5) zum Reaktivieren zumindest der Arbeitselektrode vorgesehen ist.

37. Vorrichtung nach Anspruch 36, dadurch gekennzeichnet, daß als Einrichtung zum Reaktivieren zumindest der Arbeitselektrode (13) unterhalb der Elektroden (12, 13, 14) ein Schaber oder Schleifer (5) zum Reinigen der Elektrodenoberfläche vorgesehen ist.
